# EUROPÄISCHE PATENTSCHRIFT

(11) **EP 2 428 244 B1**
(45) Veröffentlichungstag und Bekanntmachung des Hinweises auf die Patenterteilung: **20.06.2018**
(21) Anmeldenummer: 11180080.1
(22) Anmeldetag: 05.09.2011
(51) Int. Cl.: A61M 16/06, A61M 16/08

(54) **Beatmungsmaske**
Breathing mask
Masque respiratoire

(30) Priorität: 14.09.2010 DE 102010040769
(43) Veröffentlichungstag der Anmeldung: 14.03.2012
(73) Patentinhaber: MEDIN MEDICAL INNOVATIONS GMBH, 82140 Olching (DE)
(72) Erfinder: Kniewasser, Gert, 86926 Greifenberg (DE)
(74) Vertreter: Isarpatent

(56) Entgegenhaltungen:
- WO-A1-2005/123166
- DE-A1-102008 005 020
- FR-A- 832 864
- US-A- 5 577 693
- US-A1- 2005 165 334
- US-A1- 2006 032 500

## Beschreibung

Die vorliegende Erfindung betrifft eine Beatmungsmaske zum künstlichen Beatmen eines Patienten, insbesondere eine Beatmungsmaske für Frühgeborene und Kleinkinder.

Aus der US 2006/032500 A1 ist eine Beatmungsmaske bekannt, welche einen Steckanschluss zur Verbindung einer Sauerstoffleitung aufweist. Der Steckanschluss umfasst ebenfalls eine Einrichtung zum Evakuieren des durch den Patienten ausgeatmeten Gases.

Die US 2005/165334 A1 und die FR 32 864 A offenbaren Anschlussstücke für eine Beatmungsmaske, wobei das Anschlussstück eine kugelgelenkartige Verbindung aufweist.

WO 2005123166 A1 offenbart eine Atemmaskenanordnung mit einem Rahmen, einer Ellenbogenanordnung, einem an dem Rahmen vorgesehenes Polster, einem das Gesicht berührenden Abschnitt, der ein elastomeres Material aufweist, und einem Abschnitt, der eine Atmungskammer bildet.

DE 10 2008 005020 A1 offenbart eine Vorrichtung zur Beatmung, bestehend aus einem Patienteninterface und Befestigungselementen zur Positionierung der Vorrichtung am Kopf des Patienten und einem Anschluss für einen Beatmungsschlauch zur Zufuhr von Atemgas, wobei im Bereich der Vorrichtung zur Beatmung ein steuerbares Ventil angeordnet ist.

Beatmungsmasken werden unter Anderem zur künstlichen Beatmung in Intensivstationen verwendet. Diese Beatmungsmasken sind in unterschiedlichen Größen und Formen für die typischen Nasenformen ausgebildet, so dass im Wesentlichen für jede Nasenform eine formschlüssige Beatmungsmaske verfügbar ist. Die Beatmungsmaske weist einen Anschlussbereich zum Anschließen an eine Beatmungspumpe auf. Ein T-förmiges Adapterstück verbindet den Anschlussbereich mit zwei Schläuchen der Beatmungspumpe zum Zuführen und Abführen von Beatmungsgasen. Die Beatmungspumpe ist permanent aktiv, so dass ein fortwährender Luftstrom durch die beiden Schläuche fließt.

Der Anschlussschlauch zwischen dem Anschlussbereich der Beatmungsmaske und der Verzweigung des T-förmigen Adapterstücks wird durch die aktive Atmung eines Patienten gespült. Beim Einatmen fließt Luft von der Beatmungspumpe durch den Anschlussschlauch in die Beatmungsmaske. Beim Ausatmen presst die Lunge die verbrauchte Luft durch den Anschlussschlauch in Richtung zu der Beatmungspumpe gegen den Luftdruck der Beatmungspumpe, wo sie durch den fortwährenden Luftstrom mitgerissen wird.

Bei derartigen Beatmungsmasken atmet der Patient beim Einatmen die nach dem letzten Ausatmen in dem Anschlussschlauch verbliebene verbrauchte Luft wieder mit ein. Bei Frühgeborenen und auch bei Kleinkindern ist das Lungenvolumen gering, wie auch bei Patienten mit Lungenschäden. Der Anteil von eingeatmeter verbrauchter Luft ist bei solchen Patienten nicht vernachlässigbar. Der drohende Sauerstoffmangel wird durch Erhöhen des Sauerstoffanteils in den Beatmungsgasen kompensiert.

Die Verwendung einer derartigen Beatmungsmaske erfordert daher ein Bestimmen des Lungenvolumens des Patienten, um den Sauerstoffanteil individuell im Hinblick auf die verbrauchte Luft in dem Anschlussschlauch anzupassen. Die Anwendung derartiger Beatmungsmasken ist daher sehr aufwändig.

Ferner sind bei derartigen Beatmungsmasken der Anschlussbereich, das T-förmige Adapterstück und die Schläuche starr miteinander verbunden. Bei einer Bewegung des Patienten kann eine zugbelastung auf die Schläuche aufgebracht werde, wodurch die Beatmungsmaske nicht mehr dicht an der Nase des Patienten anliegt. Die Funktionalität der Beatmungsmaske ist dann nicht mehr gewährleistet.

Hiervon ausgehend ist es Aufgabe der vorliegenden Erfindung, eine verbesserte Beatmungsmaske zur Verfügung zu stellen, welche die vorgenannten Nachteile beseitigt.

Diese Aufgabe wird gelöst durch eine Beatmungsmaske mit den Merkmalen des Patentanspruchs 1.

Die Beatmungsmaske weist eine Vorkammer und einen mit der Vorkammer über eine Gasaustauschöffnung verbundenen Nasenaufnahmeraum auf. Diese Vorkammer kann durch die von einer Gasspüleinrichtung, beispielsweise einer Beatmungspumpe, bereitgestellten Gase in einer typischen Anwendung permanent gespült werden. Der geringe Abstand der Vorkammer zu dem Nasenaufnahmeraum, welche mittels der Gasaustauschöffnung miteinander verbunden sind, und damit zur Nase eines Patienten verringert das Volumen an verbrauchtem Atemgas auf einen nahezu vernachlässigbaren Wert. Aufgrund der Verschwenkbarkeit der Gelenkelemente zueinander können der Gasauslass und der Gaseinlass einer Bewegung des Patienten folgen, wodurch Verbindungsschläuche zwischen der Beatmungsmaske und der Gasspüleinrichtung stets zugfrei sind. Hierdurch ist immer ein sicherer Sitz der Beatmungsmaske auf der Nase des Patienten gewährleistet.

Vorteilhafte Ausgestaltungen und Weiterbildungen der Beatmungsmaske sind in den Unteransprüchen und den Ausführungsformen angegeben.

Erfindungsgemäß stehen die Gelenkelemente derart miteinander in Wirkeingriff, dass diese ein bewegliches Gelenk, insbesondere ein Kugelgelenk, der Beatmungsmaske ausbilden. Hierdurch können Verbindungsschläuche zwischen der Beatmungsmaske und der Gasspüleinrichtung spannungsfrei verlegt werden, wodurch die Zuverlässigkeit der Beatmungsmaske erhöht wird.

Erfindungsgemäß umschließen die Gelenkelemente die Vorkammer zumindest abschnittsweise, wodurch ein Gasvolumen der Vorkammer vorteilhaft mittels der Gelenkelemente definiert ist.

Gemäß einer bevorzugten Ausführungsform der Beatmungsmaske ist die Vorkammer kugelförmig, oder ellipsoidförmig, wodurch gekrümmte oder ellipsoidförmige innere Flächen der Vorkammer von einströmendem Gas vollständig gespült werden.

Gemäß einer weiter bevorzugten Ausführungsform der Beatmungsmaske ist die Vorkammer zylinderförmig und die Gasaustauschöffnung sowie der Gaseinlass und der Gasauslass sind jeweils an einer Stirnfläche der zylinderförmigen Vorkammer angeordnet. Hierdurch ist ein besonders einfacher konstruktiver Aufbau der Beatmungsmaske verwirklicht, wodurch die Herstellungskosten der Beatmungsmaske reduziert werden.

Erfindungsgemäß ist eines der Gelenkelemente in dem anderen Gelenkelement beweglich eingesetzt. Mittels eines relativen Bewegens der zwei Gelenkelemente zueinander kann die Lage des Gasauslasses und des Gaseinlasses vorteilhaft relativ zu dem Nasenaufnahmeraum justiert werden, wodurch sich der mögliche Einsatzbereich der Beatmungsmaske erweitert.

Erfindungsgemäß ist eines der Gelenkelemente einstückig mit dem Nasenaufnahmeraum verbunden. Ein ungewolltes oder gewaltsames Ablösen dieses Gelenkelementes von dem Nasenaufnahmeraum ist nicht möglich, wodurch die Betriebssicherheit der Beatmungsmaske erhöht wird.

Gemäß einer weiter bevorzugten Ausführungsform der Beatmungsmaske umschließt der Nasenaufnahmeraum die Nase zumindest abschnittsweise formschlüssig. Wodurch eine gute Abdichtung zwischen der Beatmungsmaske und der Nase des Patienten erreicht wird.

Erfindungsgemäß grenzen die Vorkammer und der Nasenaufnahmeraum jeweils an die Gasaustauschöffnung unmittelbar an. Hierdurch wird zuverlässig die Ansammlung von verbrauchter Atemluft in der Atemmaske unterbunden. Erfindungsgemäß weist die Vorkammer ein von dem Nasenaufnahmeraum abgetrenntes Volumen auf. Die Vorkammer und der Nasenaufnahmeraum sind durch die Gasaustauschöffnung miteinander verbunden. Die Vorkammer ermöglicht den Aufbau eines Gegendrucks gegen das Ausatmen des Patienten und den Abtransport verbrauchter Atemluft. Erfindungsgemäß ist ein Durchmesser der Gasaustauschöffnung geringer als ein Durchmesser der Vorkammer. Der Durchmesser der Vorkammer bezeichnet den größtmöglichen inneren Durchmesser der Vorkammer in einer Ebene parallel zu der Öffnung. Durch den geringeren Durchmesser der Gasaustauschöffnung als der Durchmesser der Vorkammer ist vorteilhaft ein abgetrenntes Volumen der Vorkammer ausgebildet. Erfindungsgemäß ist die Vorkammer in Richtung zu dem Nasenaufnahmeraum verjüngt ausgebildet. Hierdurch ergibt sich eine konstruktive Abgrenzung der Vorkammer zu dem Nasenaufnahmeraum.

Gemäß einer weiter bevorzugten Ausführungsform der Beatmungsmaske weist diese zum Befestigen derselben an dem Patienten eine Befestigungslasche auf, wodurch eine gute Fixierung der Atemmaske ohne zusätzliche Hilfsmittel, wie beispielsweise Klebebändern, an dem Patienten gewährleistet ist. Dies vereinfacht die Anwendung der Beatmungsmaske.

Die vorliegende Erfindung wird nachfolgend anhand bevorzugter Ausführungsformen und der beiliegenden Zeichnung erläutert. In der Zeichnung zeigen:
- Fig. 1: eine perspektivische Ansicht einer bevorzugten Ausführungsform einer Beatmungsmaske;
- Fig. 2: eine Schnittansicht der Beatmungsmaske nach Fig. 1;
- Fig. 3: eine weitere Schnittansicht der Beatmungsmaske gemäß der Schnittlinie A-A nach Fig. 2;
- Fig. 4: eine Unteransicht der Beatmungsmaske nach Fig. 1;
- Fig. 5: eine Seitenansicht einer weiteren bevorzugten Ausführungsform einer Beatmungsmaske;
- Fig. 6: eine perspektivische Ansicht einer noch weiteren bevorzugten Ausführungsform einer Beatmungsmaske;
- Fig. 7: eine Untenansicht der bevorzugten Ausführungsform der Beatmungsmaske gemäß Fig. 6;
- Fig. 8: eine Schnittansicht der bevorzugten Ausführungsform der Beatmungsmaske gemäß der Schnittlinie C-C nach Fig. 7;
- Fig. 9: eine perspektivische Ansicht einer noch weiteren bevorzugten Ausführungsform einer Beatmungsmaske;
- Fig. 10: eine Schnittansicht der Beatmungsmaske gemäß der Fig. 9;
- Fig. 11: eine weitere Schnittansicht der Beatmungsmaske gemäß der Schnittlinie D-D der Fig. 10;
- Fig. 12: eine noch weitere Schnittansicht der Beatmungsmaske gemäß der Schnittlinie F-F der Fig. 11; und
- Fig. 13: eine noch weitere Schnittansicht der Beatmungsmaske gemäß der Schnittlinie G-G der Fig. 11.

In den Figuren bezeichnen gleiche Bezugszeichen gleiche oder funktionsgleiche Elemente.

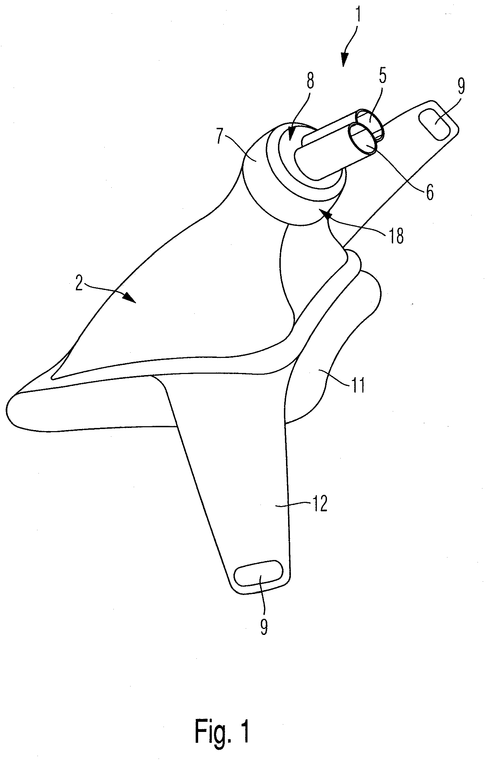

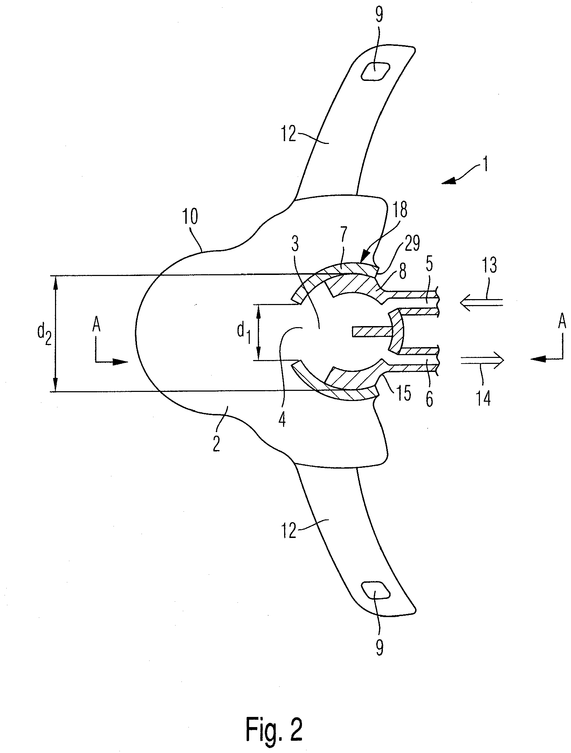

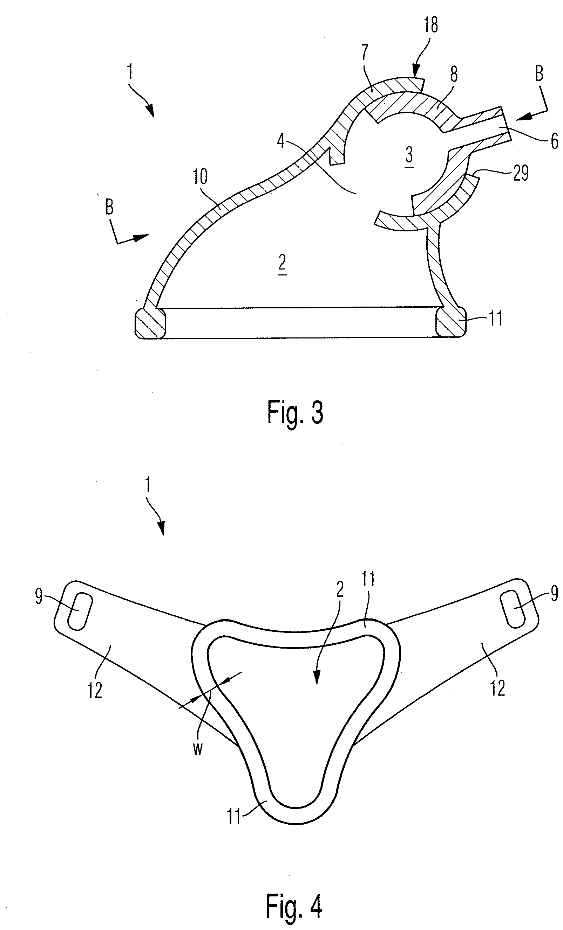

Eine erste bevorzugte Ausführungsform einer Beatmungsmaske 1 ist in unterschiedlichen Ansichten in den Figuren 1 bis 4, auf die im Folgenden gleichzeitig Bezug genommen wird, gezeigt. Eine perspektivische Ansicht der Beatmungsmaske 1 ist in Fig. 1 illustriert. Die Fig. 2 zeigt einen horizontalen Schnitt durch die Beatmungsmaske 1. Fig. 3 zeigt einen vertikalen Schnitt entlang der Schnittlinie A-A gemäß Fig. 2. Eine Schnittebene des horizontalen Schnitts der Beatmungsmaske 1 nach Fig. 2 ist in Fig. 3 mittels der Schnittlinie B-B angedeutet. In Fig. 4 ist eine Ansicht der Beatmungsmaske 1 von unten illustriert.

Die Beatmungsmaske 1 weist einen Nasenaufnahmeraum 2 auf. Eine geometrische Form und ein Volumen des Nasenaufnahmeraums 2 sind derart ausgebildet, dass beim Aufsetzen der Beatmungsmaske 1 auf das Gesicht eines Patienten der Nasenaufnahmeraum 2 vorzugsweise vollständig oder nahezu vollständig von der Nase ausgefüllt ist. Zugleich sollte zweckmäßigerweise auch vermieden werden, dass eine Wandung 10 des Nasenaufnahmeraums 2 auf die Nase drückt. Hierzu wird die Beatmungsmasken 1 beispielsweise in unterschiedlichen Formen und Größen bereitgestellt. Zweckmäßigerweise kann auch das Material für die Wandung 10 mit einem weichen und nachgiebigen Material hergestellt sein. Insbesondere eignen sich als Material für die Wandung 10 weiche Silikon- und/oder Polyurethankunststoffe.

Ein unterer Rand des Nasenaufnahmeraums 2 weist einen verdickten Saum 11 auf. Eine Wandstärke w des Saums 11 kann im Bereich von 4 mm liegen. Die Wandstärke w des Saums 11 ist vorzugsweise größer als eine Wandstärke der Wandung 10. Durch die hierdurch gewährleistete breite Auflagefläche des Saums 11 auf dem Gesicht werden Druckstellen vermieden. Das Material des breiten Saums 11 kann gleich dem Material der Wandung 10 des Nasenaufnahmeraums 2 sein. Der Saum 11 kann auch ein anderes Material als die Wandung 10 aufweisen. Beispielsweise kann der Saum 11 mit einer umlaufenden Nut versehen sein, welche zur Aufnahme eines Dichtmittels, beispielsweise eines Silikongels, ausgebildet ist. Mittels des Silikongels ist eine sichere Abdichtung der Beatmungsmaske 1 zu dem Gesicht des Patienten gewährleistet.

Eine Fixierung der Beatmungsmaske 1 kann über Laschen 12 erfolgen. Die Laschen 12 können im Bereich des breiten Saums 11 befestigt sein. Die Anzahl der Laschen 12 ist beliebig, beispielsweise weist die Beatmungsmaske zwei oder drei Laschen 12 auf. Vorzugsweise weist jede Lasche 12 eine Öffnung 9 zum Durchführen eines Befestigungsmittels wie eines Befestigungsbandes auf.

Beatmungsgase werden über eine Öffnung 4, insbesondere eine Gasaustauschöffnung 4, in den Nasenaufnahmeraum 2 eingebracht. Durch die Gasaustauschöffnung 4 werden auch vom Patienten ausgeatmete Gase abgeführt. Die Öffnung 4 befindet sich vorzugsweise in einem Abschnitt des Nasenaufnahmeraums 2, der zur Aufnahme der Nasenspitze ausgebildet ist.

An die Gasaustauschöffnung 4 angrenzend ist eine Vorkammer 3 der Beatmungsmaske 1 ausgebildet. Die Vorkammer 3 ist vorzugsweise strukturell von dem Nasenaufnahmeraum 2 abgetrennt und bildet ein von dem Nasenaufnahmeraum 2 getrenntes Volumen aus. Eine Verbindung zwischen der Vorkammer 3 und dem Nasenaufnahmeraum 2 wird durch die Öffnung 4 bereitgestellt. Die Gasaustauschöffnung 4 ist vorzugsweise zum Austausch von Gasen zwischen dem Nasenaufnahmeraum 2 und der Vorkammer 3 der Beatmungsmaske 1 ausgebildet. Vorzugsweise und in der dargestellten Ausführungsform gezeigt befindet sich zwischen dem Nasenaufnahmeraum 2 und der Vorkammer 3 kein Verbindungsstück, - Rohr oder -Schlauch. Die Öffnung 4 zu dem Nasenaufnahmeraum 2 bildet somit zugleich die Öffnung der Vorkammer 3. Die Öffnung 4 kann einen kreisrunden oder einen beliebigen Querschnitt aufweisen.

Die Öffnung 4 weist beispielsweise einen Querschnitt oder einen Durchmesser d1 auf, der geringer als ein Querschnitt oder ein Durchmesser d2 der Vorkammer 3 ist. Der Durchmesser d2 der Vorkammer 3 wird vorzugsweise in einer Ebene parallel zu der Öffnung 4 und an einer breitesten Stelle der Vorkammer 3 bestimmt. Das heißt, die Vorkammer 3 verjüngt sich zu der Öffnung 4 hin oder die Öffnung 4 weist einen geringeren Durchmesser als eine Stirnfläche der Vorkammer 3 auf. Hierdurch ergibt sich eine strukturelle Abgrenzung der Vorkammer 3 von dem Nasenaufnahmeraum 2. Im Gegensatz zu der Vorkammer 3 steht ein Schlauch oder ein Rohrstück, dessen Öffnung den gleichen Querschnitt wie der Querschnitt des Rohrs aufweist. Die Vorkammer 3 weist vorzugsweise eine zumindest abschnittsweise kugelförmige, verrundete, elipsoidförmige und/oder zylinderförmige Innengeometrie auf.

Die Vorkammer 3 weist einen Anschluss 5 zum Einlassen von Gas und einen Anschluss 6 zum Auslassen von Gas auf. An die beiden Anschlüsse 5, 6 kann eine Gasspüleinrichtung, insbesondere eine externe Beatmungspumpe, angeschlossen werden. Beispielsweise wird der Gaseinlass 5 der Beatmungsmaske 1 an einen Inspirationsanschluss und der Gasauslass 6 an einen Expirationsanschluss der Gasspüleinrichtung angeschlossen. Ein einströmendes Beatmungsgas 13 strömt in die Vorkammern 3 ein und als ausströmendes Gas 14 wieder aus dieser aus. Das ausströmende Gas 14 ist mit dem Ausatemgas des Patienten angereichert. Die permanente Spülung der Vorkammer 3 bewirkt, dass sich hierin kein verbrauchtes Atemgas sammeln kann. In dem Ausatemgas des Patienten angereichertes Kohlendioxid wird aus der Beatmungsmaske 1 so vollständig ausgewaschen. Ein an dem Gaseinlass 5 eingestellter Gasdruck wird derart eingestellt, dass ein ausreichender Gasstrom gewährleistet wird und das Atmen des Patienten nicht erschwert wird. Der Patient atmet gegen den Gasdruck und das von dem Patienten ausgeatmete Atemgas wird mittels des kontinuierlichen Gasstroms der Gasspüleinrichtung über den Gasauslass 6 aus der Atemmaske 1 entfernt. Von dem Patienten verbrauchte Luft kann von diesem daher nicht wieder eingeatmet werden.

Der Gaseinlass 5 und der Gasauslass 6 können parallel zueinander, wie in Fig. 2 angedeutet, angeordnet sein. Zudem kann optional in der Vorkammer 3 ein Zwischensteg 15 zwischen dem Einlass 5 und dem Auslass 6 angeordnet werden. Hierdurch kann eine Führung des einströmenden Gases 13 und/oder des ausströmenden Gases 14 in der Vorkammer 3 hinsichtlich der Spülungseigenschaften optimiert werden. Der Gaseinlass 5 und/oder der Gasauslass 6 können als sich aus der Beatmungsmaske 1 erstreckende Rohrstücke ausgebildet sein.

In einer nicht dargestellten Ausführungsform der Beatmungsmaske kann die Vorkammer 3 einstückig ausgebildet sein. Dabei ist es insbesondere vorteilhaft, den Nasenaufnahmeraum 2 mit der Vorkammer 3 einstückig zu fertigen.

In der in den Fig. 1 bis 4 dargestellten bevorzugten Ausführungsform der Beatmungsmaske 1 ist die Vorkammer 3 mehrteilig, insbesondere zweiteilig, ausgebildet. Die Vorkammer 3 weist vorzugsweise ein als äußere Schale 7 der Vorkammer 3 ausgebildetes erstes Gelenkelement 7 auf, welches mit der Wandung 10 des Nasenaufnahmeraums 2 vorzugsweise mechanisch verbunden ist. Zweckmäßigerweise sind die Wandung 10 und die äußere Schale 7 der Vorkammer 3 einstückig ausgebildet. Die äußere Schale 7 weist in einer bevorzugten Ausgestaltung die Form eines Kugelsegments oder eines ellipsoidförmigen Körpers auf. Die äußere Schale 7 weist insbesondere die Gasaustauschöffnung 4 auf.

Die Vorkammer 3 weist ferner ein als innere Schale 8 ausgebildetes zweites Gelenkelement 8 auf. Das zweite Gelenkelement 8 ist vorzugsweise in dem ersten Gelenkelement 7 zumindest abschnittsweise aufgenommen und relativ zu diesem verschwenkbar gelagert, wodurch die Gelenkelemente 7, 8 ein Gelenk 18, insbesondere ein Kugelgelenk 18, der Beatmungsmaske 1 ausbilden. Die innere Schale 8 weist vorzugsweise die Form eines Kugelsegments oder eines ellipsoidförmigen Körpers auf. Hierdurch ergibt sich eine verrundete Innengeometrie der Vorkammer 3, wodurch das Ausspülen von verbrauchter Atemluft aus der Vorkammer 3 aufgrund eines verringerten Strömungswiderstandes vereinfacht und verbessert wird. Die innere Schale 8 ist vorzugsweise formschlüssig mit der äußeren Schale 7 in Wirkverbindung, um ein Austreten von Gas zwischen den Schalen 7, 8 zu unterbinden. Insbesondere umschließen die Schalen 7, 8 die Vorkammer 3 zumindest abschnittsweise. Der Gaseinlass 5 und der Gasauslass 6 sind vorzugsweise an der inneren Schale 8 angeordnet. Die äußere Schale 7 weist ferner einen Durchbruch 29 auf, durch welchen der Gaseinlass 5 und der Gasauslass 6 geführt sind. Im Bereich des Durchbruchs 29 liegt die innere Schale 8 zu der Umgebung frei. Ein Querschnitt des Durchbruchs 29 ist vorzugsweise derart gewählt, dass die Verschwenkbarkeit des Gelenkes 18 nicht begrenzt oder nur in einem gewünschten Umfang begrenzt ist. Der Querschnitt des Durchbruchs 29 ist vorzugsweise kleiner gewählt als der Durchmesser d2 der Vorkammer. Die bewegliche Lagerung der inneren Schale 8 in der äußeren Schale 7 ermöglicht es, eine Ausrichtung des Gaseinlasses 5 und des Gasauslasses 6 bezüglich des Nasenaufnahmeraums 2 mittels des Gelenkes 18 einzustellen. Hierdurch kann beispielsweise eine bei einer Bewegung des Patienten auf die Verbindungsschläuche zwischen der Beatmungsmaske 1 und der Gasspüleinrichtung aufgebrachte Zugbelastung ein Verstellen des Gelenkes 18 bewirken, wodurch die Verbindungsschläuche stets zugfrei sind und ein sicherer Sitz der Beatmungsmaske auf dem Gesicht des Patienten gewährleistet ist.

Die innere Schale 8 kann aus dem gleichen Material wie die äußere Schale 7 hergestellt werden. Die Materialien für die innere Schale 8 und die äußere Schale 7 können unterschiedlich gewählt werden. Beispielsweise kann die innere Schale 8 mit einem harten Werkstoff, insbesondere einem formstabilen harten Kunststoff, hergestellt werden. Hierbei ist es möglich, die äußere Schale 7 mit einem elastischen Material, insbesondere einem elastischen Kunststoff, herzustellen. Die äußere Schale 7 ist in ihrer Form beispielsweise derart ausgebildet, dass sie durch die eingesetzte innere Schale 8 vorzugsweise gedehnt wird. Hierdurch kann eine formschlüssige Wirkverbindung der äußeren Schale 7 und der inneren Schale 8 gewährleistet werden. Ein Austreten von Gas zwischen den Schalen 7, 8 kann folglich auf diese Weise zuverlässig vermieden werden und eine zusätzliche Dichteinrichtung zur gasdichten Abdichtung der Schalen 7, 8 zueinander ist verzichtbar. Die Verwendung eines harten Werkstoffes für die innere Schale 8 kann unter anderem den Vorteil aufweisen, dass ein Aufstecken von Anschlüssen auf den Gaseinlass 5 und/oder den Gasauslass 6 vereinfacht wird. Alternativ kann die äußere Schale 7 mit einem harten Werkstoff und die innere Schale 8 mit einem weichen Werkstoff gebildet sein.

Die Vorkammer 3 kann auch mittels eines zylindrisch ausgebildeten Volumens gebildet werden. Hierbei ist es zweckmäßig, den Einlass und den Auslass 5, 6 sowie die Öffnung 4 an Stirnflächen der Zylinderform anzuordnen.

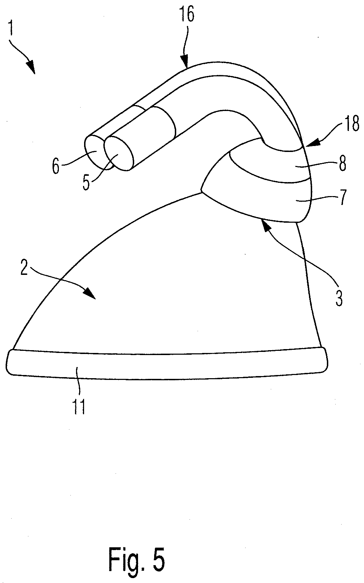

Eine weitere bevorzugte Ausführungsform der Beatmungsmaske 1 ist in Fig. 5 illustriert. Die Ausführungsform der Beatmungsmaske 1 gemäß der Fig. 5 unterscheidet sich von der Ausführungsform der Beatmungsmaske 1 gemäß der Fig. 1 bis 4 lediglich durch das Vorsehen eines U-förmigen oder gewinkelten Verlängerungsstückes 16, welches an dem Gaseinlass 5 und/oder dem Gasauslass 6 angeordnet ist. Das Verlängerungsstück 16 kann mit dem Einlass 5 und/oder dem Auslass 6 unlösbar verbunden sein. Das Verlängerungsstück 16 ermöglicht beispielsweise eine Umleitung der Gasströme 13, 14 um etwa 180°, wodurch die Verbindungsschläuche zur Gasspüleinrichtung beispielsweise über den Kopf des Patienten, weg von dem Mundbereich des Patienten, geführt werden können.

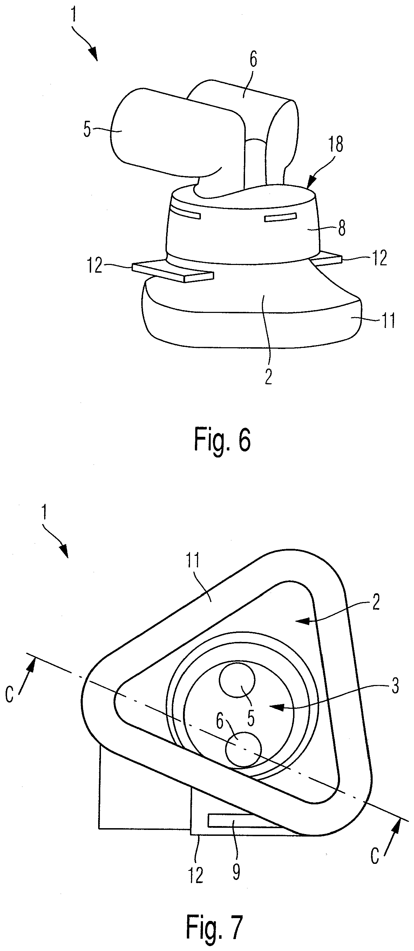

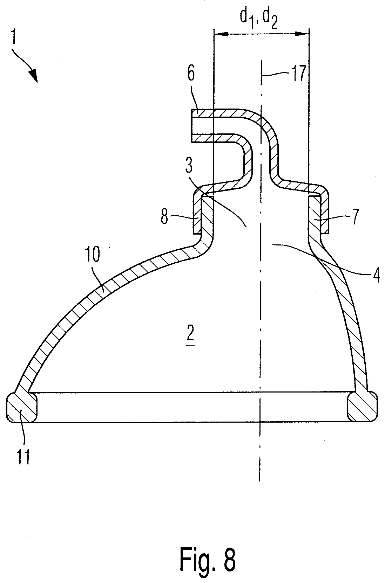

Eine weitere bevorzugte Ausführungsform einer Beatmungsmaske 1 illustrieren die Fig. 7 bis 8, auf die im Folgenden gleichzeitig Bezug genommen wird. Die Ausführungsform der Beatmungsmaske 1 gemäß der Fig. 7 bis 9 unterscheidet sich von der Ausführungsform der Beatmungsmaske 1 gemäß den Fig. 1 bis 4 lediglich durch die Ausgestaltung der Gelenkelemente 7, 8. Die Beatmungsmaske 1 weist einen Nasenaufnahmeraum 2 und einen über eine Öffnung 4, insbesondere eine Gasaustauschöffnung 4, mit dem Nasenaufnahmeraum 2 verbundene Vorkammer 3 auf. Der Nasenaufnahmeraum 2 dient vorzugsweise zum zumindest abschnittsweisen formschlüssigen Aufnehmen der Nase eines Patienten. Das erste Gelenkelement 7, welches vorzugsweise als hohlzylinderförmige Schale 7 ausgebildet ist, ist vorzugsweise integral mit einer Wandung 10 des Nasenaufnahmeraums 2 verbunden, wobei das erste Gelenkelement 7 insbesondere die Öffnung 4 aufweist.

Die Vorkammer 3 weist eine zylindrische Form auf, wobei die Öffnung 4 an einer Stirnfläche der Zylinderform angeordnet ist. Abweichend zu den zuvor beschriebenen Ausführungsformen der Beatmungsmaske 1 gemäß den Fig. 1 bis 4 sowie der Fig. 5 ist ein zweites Gelenkelement 8, welches vorzugsweise als hohlzylinderförmige Schale 8 ausgebildet ist, nicht innerhalb des ersten Gelenkelementes 7 angeordnet, sondern außerhalb des ersten Gelenkelementes 7. Das zweite Gelenkelement 8 umfasst das erste Gelenkelement 7 zumindest abschnittsweise. Die Gelenkelemente 7, 8 stehen vorzugsweise über zylindrische Mantelflächen miteinander in Wirkverbindung. Das zweite Gelenkelement 8 weist vorzugsweise einen Gaseinlass 5 und einen Gasauslass 6 zum jeweiligen Anschließen an eine Gasspüleinrichtung auf. Der Gaseinlass 5 und der Gasauslass 6 sind vorzugsweise an einer der mit der Öffnung 4 versehenen Stirnfläche der Zylinderform der Vorkammer 3 angeordnet.

Eine innere Mantelfläche des zweiten Gelenkelementes 8 umschließt das erste Gelenkelement 7 vorzugsweise derart, dass die beiden Gelenkelemente 7, 8 ein gasdichtes und um eine Mittelachse 17 verschwenkbares Gelenk 18 der Beatmungsmaske 1 bilden. Das zweite Gelenkelement 8 kann somit gegenüber dem Nasenaufnahmeraum 2 verdreht werden. Dies ermöglicht es, den an dem zweiten Gelenkelement 8 angebrachten Einlass 5 und Auslass 6 so bezüglich des Nasenaufnahmeraums 2 zu verdrehen, dass eine spannungsfreie Verbindung mit der Gasspüleinrichtung mittels Anschlussschläuchen möglich ist. Ein innerer Durchmesser d2 der Vorkammer 3 ist in diesem bevorzugten Ausführungsbeispiel der Beatmungsmaske 1 in etwa gleich groß wie ein Durchmesser d1 der die Vorkammer 3 und den Nasenaufnahmeraum 2 verbindenden Gasaustauschöffnung 4. Das zweite Gelenkelement 8 kann einen Steg aufweisen, welcher zwischen dem Gaseinlass 5 und dem Gasauslass 6 angeordnet ist.

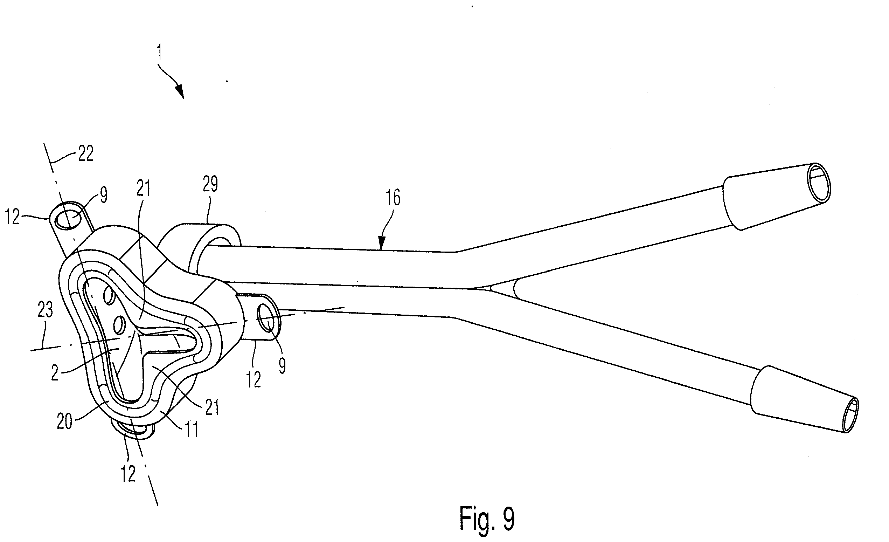

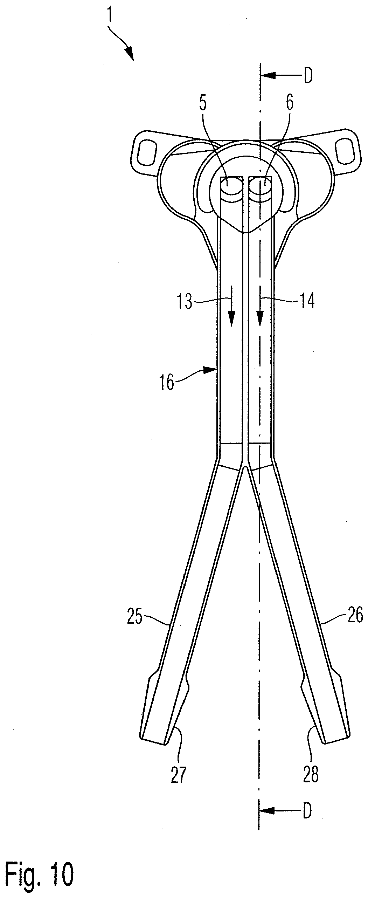

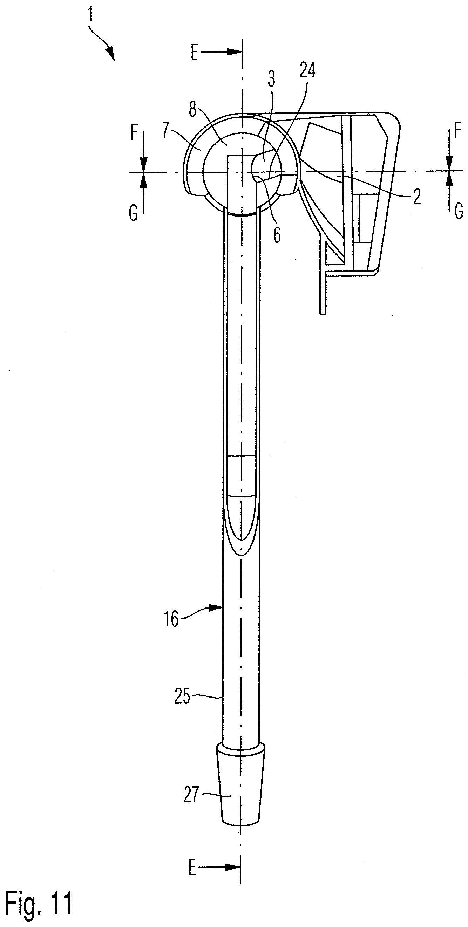

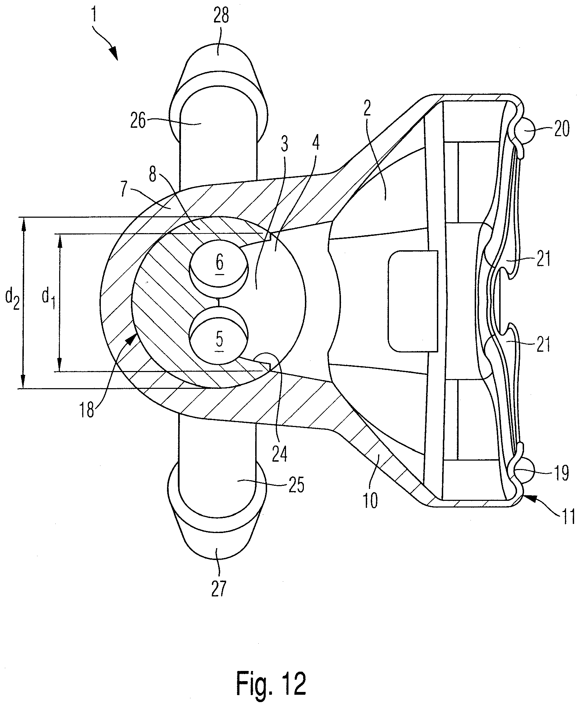

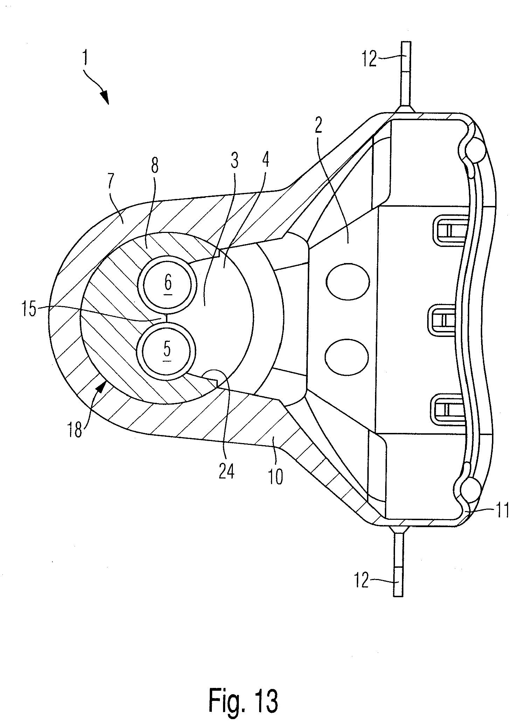

Die Fig. 9 bis 13, auf die im Folgenden gleichzeitig Bezug genommen wird, illustrieren ein noch weiteres bevorzugtes Ausführungsbeispiel einer Beatmungsmaske 1. Die Fig. 9 illustriert die Beatmungsmaske 1 in einer perspektivischen Ansicht, die Fig. 10 stellt die Beatmungsmaske 1 in einer Schnittansicht dar, die Fig. 11 zeigt eine weitere Schnittansicht der Beatmungsmaske 1 gemäß der Schnittlinie D-D der Fig. 10, wobei in der Fig. 11 die Schnittlinie E-E den Schnittverlauf der Fig. 10 andeutet. Die Fig. 12 und 13 zeigen weitere Schnittansichten der Beatmungsmaske 1 gemäß der Schnittlinien F-F und G-G der Fig. 11.

Die Beatmungsmaske 1 weist vorzugsweise einen Nasenaufnahmeraum 2 auf. Der Nasenaufnahmeraum 2 ist zum zumindest abschnittsweise formschlüssigen Aufnehmen der Nase eines Patienten ausgebildet. Eine Wandung 10 des Nasenaufnahmeraums 2 ist beispielsweise derart ausgebildet, dass die Beatmungsmaske 1 möglichst eng an der Nase des Patienten anliegt. Ein umlaufender Saum 11 des Nasenaufnahmeraums 2 verläuft vorzugsweise in etwa senkrecht zu der Wandung 10 und ragt zumindest abschnittsweise in den Nasenaufnahmeraum 2 hinein. Der Saum 11 ist vorzugsweise integraler Bestandteil der Wandung 10. Die Wandung 10 und/oder der Saum 11 sind vorzugsweise mit einem flexiblen, weichen Material gebildet. Der Saum 11 und die Wandung 10 können mit dem gleichen oder mit unterschiedlichen Materialien gebildet sein. Der Saum 11 dient der Vergrößerung der Auflagefläche der Beatmungsmaske 1 auf dem Gesicht des Patienten, wodurch sich eine verbesserte Abdichtung der Beatmungsmaske 1 gegen das Gesicht und ein erhöhter Tragekomfort für den Patienten ergeben.

Der Saum 11 weist vorzugsweise eine um den Nasenaufnahmeraum 2 umlaufende Nut 19 auf, welche vorzugsweise mit einem Dichtmittel 20, insbesondere mit einem Silikongel 20, zum Abdichten der Beatmungsmaske 1 gegen das Gesicht des Patienten ausgebildet ist. Der Saum 11 weist weiterhin vorzugsweise Dichtlappen 21 auf, welche beispielsweise mit einem besonders flexiblen Material zur Anlage an der Nase des Patienten ausgebildet sind. Insbesondere schließen die Dichtlappen 21 zumindest abschnittsweise den Nasenaufnahmeraum 2 zur Umgebung hin ab. Die Dichtlappen 21 sind vorzugsweise einstückig mit dem Saum 11 ausgebildet.

Vorzugsweise weist der gesamte Nasenaufnahmeraum 2 ein flexibles Material auf. Die Beatmungsmaske 1 weist weiterhin an einer Außenseite des Nasenaufnahmeraums 2 angeordnete Befestigungslaschen 12 zur Befestigung der Beatmungsmaske 1 an dem Kopf eines Patienten auf. Vorzugsweise weist die Beatmungsmaske 1 drei Befestigungslaschen 12 auf. Vorzugsweise zwei der Befestigungslaschen 12 sind auf einer gemeinsamen Achse 22 angeordnet und die dritte der Befestigungslaschen 12 ist auf einer zu der Befestigungsachse 22 senkrecht stehenden Befestigungsachse 23 vorgesehen. Die Laschen 12 bilden so in etwa die Ecken eines gleichschenkeligen Dreiecks.

Die Beatmungsmaske 1 weist weiterhin einen Vorraum 3 auf, welcher über eine Öffnung 4, insbesondere eine Gasaustauschöffnung 4, mit dem Nasenaufnahmeraum 2 verbunden ist. Der Vorraum 3 ist vorzugsweise in etwa kugelförmig ausgebildet. Ein Durchmesser d1 der Öffnung 4 ist vorzugsweise kleiner als ein Durchmesser d2 des Vorraumes 3. Der Vorraum 3 ist vorzugsweise integral mit dem Nasenaufnahmeraum 2 ausgebildet. Der Vorraum 3 weist ein vorzugsweise integral mit der Wandung 10 als äußere Schale 7 ausgebildetes erstes bzw. äußeres Gelenkelement 7 auf und ein in dem äußeren Gelenkelement 7 aufgenommenes zweites Gelenkelement 8 oder inneres Gelenkelement 8. Das innere Gelenkelement 8 weist vorzugsweise in etwa die Form einer Kugel auf. Das äußere Gelenkelement 7 weist vorzugsweise die Form einer Schale 7 mit einer zumindest abschnittsweise kugelförmig, insbesondere komplementär zu einer Außengeometrie des zweiten Gelenkelementes 8, ausgebildeten Innengeometrie auf. Das zweite Gelenkelement 8 ist vorzugsweise zumindest teilweise formschlüssig in dem ersten Gelenkelement 7 aufgenommen und relativ zu diesem verschwenkbar. Die Gelenkelemente 7, 8 bilden vorzugsweise ein Gelenk 18, insbesondere ein Kugelgelenk 18, der Beatmungsmaske 1. Die Gelenkelemente 7, 8 sind dabei vorzugsweise derart in Wirkverbindung, dass das Gelenk 18 gasdicht ist. Beispielsweise ist das zweite Gelenkelement 8 aus einem flexibleren Material, beispielsweise aus einem Silikonmaterial, gebildet als das äußere Gelenkelement 7. Alternativ kann auch das äußere Gelenkelement 7 aus einem flexibleren Material gebildet sein als das innere Gelenkelement 8.

Das innere Gelenkelement 8 weist vorzugsweise einen Gaseinlass 5 und einen Gasauslass 6 auf. Über den Gaseinlass 5 wird dem Patienten frisches Beatmungsgas 13 zugeführt und über den Gasauslass wird mit Ausatemgas angereichertes ausströmendes Gas 14 abgeführt. Der Gaseinlass 5 und der Gasauslass 6 sind vorzugsweise mittels eines Durchbruchs 29 durch das äußere Gelenksegment 7 geführt. Ein Querschnitt des Durchbruchs 29 ist vorzugsweise kleiner als der Durchmesser d2 der Vorkammer 2. Im Bereich des Durchbruchs 29 liegt das innere Gelenksegment 8 vorzugsweise zur Umgebung hin frei. Vorzugsweise sind der Gaseinlass 5 und der Gasauslass 6 mit einem Verlängerungsstück 16 der Beatmungsmaske 1 verbunden. Der Gaseinlass 5 und der Gasauslass 6 liegen benachbart zueinander, wobei zwischen ihnen ein Zwischensteg 15 vorgesehen sein kann. Das Verlängerungsstück 16 ist vorzugsweise integraler Bestandteil des zweiten Gelenkelementes 8. Das Verlängerungsstück 16 ist beispielsweise in Form zweier miteinander verbundener sich aus dem zweiten Gelenkelement 8 erstreckender Rohrabschnitte 25, 26 ausgebildet. Die Rohrabschnitte 25, 26 erstrecken sich aus dem zweiten Gelenkelement 8 heraus und verlaufen zunächst parallel zueinander, um dann in Form eines Ypsilons auseinanderzulaufen. Konische Endstücke 27, 28 der Rohrabschnitte 25, 26 dienen zur einfachen Verbindung des Gaseinlasses 5 und des Gasauslasses 6 mit einer nicht dargestellten Gasspüleinrichtung.

Das zweite Gelenkelement 8 weist eine der Öffnung 4 zugewandte Ausnehmung 24 auf, welche der Verbindung des Gaseinlasses 5 und des Gasauslasses 6 mit dem Nasenaufnahmeraum 2 dient. Die Ausnehmung 24 weist vorzugsweise einen Öffnungsquerschnitt auf, welcher zumindest geringfügig kleiner ist als der Durchmesser d1 der Öffnung 4. Die Ausnehmung 24 verjüngt sich vorzugsweise von der Gasaustauschöffnung 4 hin zu dem Gasauslass 5 und dem Gaseinlass 6. Hierdurch wird vorteilhafterweise eine gute Gasdurchspülung des Vorraums 3 erreicht. Die Ausnehmung 24 bildet im Wesentlichen ein Gasvolumen des Vorraums 3 der Beatmungsmaske 1.

Das Gelenk 18 ermöglicht eine Bewegung des Verlängerungsstückes 16 relativ zu der Beatmungsmaske 1 derart, dass Verbindungsschläuche zwischen den Gaseinlass 5, dem Gasauslass 6 und der Gasspüleinrichtung spannungsfrei verlegt werden können. Bewegungen des Patienten, die eine Zugbelastung auf die Verbindungsschläuche aufbringen könnten kann das Gelenk 18 folgen, so dass die Verbindungsschläuche stets zugfrei sind und ein sicherer Sitz der Beatmungsmaske 1 gewährleistet ist.

### Bezugszeichenliste

- 1: Beatmungsmaske
- 2: Nasenaufnahmeraum
- 3: Vorraum
- 4: Öffnung
- 5: Gaseinlass
- 6: Gasauslass
- 7: Gelenkelement
- 8: Gelenkelement
- 9: Öffnung
- 10: Wandung
- 11: Saum
- 12: Lasche
- 13: Beatmungsgas
- 14: ausströmendes Gas
- 15: Zwischensteg
- 16: Verlängerungsstück
- 17: Mittelachse
- 18: Gelenk
- 19: Nut
- 20: Dichtmittel
- 21: Dichtlappen
- 22: Achse
- 23: Achse
- 24: Ausnehmung
- 25: Rohrabschnitt
- 26: Rohrabschnitt
- 27: Endstück
- 28: Endstück
- 29: Durchbruch
- d1: Durchmesser
- d2: Durchmesser
- w: Wandstärke

## Patentansprüche

1. Beatmungsmaske (1) zum künstlichen Beatmen eines Patienten, mit:
einem Nasenaufnahmeraum (2) zum zumindest abschnittsweisen Aufnehmen der Nase des Patienten; und
einer mittels einer Gasaustauschöffnung (4) mit dem Nasenaufnahmeraum (2) verbundenen Vorkammer (3), welche ein erstes Gelenkelement (7) und ein mit dem ersten Gelenkelement (7) in Wirkverbindung stehendes, relativ zu dem ersten Gelenkelement (7) verschwenkbares, zweites Gelenkelement (8) mit einem Gaseinlass (5) und einem Gasauslass (6) zum jeweiligen Anschließen an eine Gasspüleinrichtung, aufweist;
wobei eines der Gelenkelemente (7, 8) in dem anderen Gelenkelement (7, 8) abschnittsweise aufgenommen und beweglich eingesetzt ist, wodurch die Gelenkelemente (7, 8) derart miteinander in Wirkeingriff stehen, dass diese ein bewegliches Gelenk (18) der Beatmungsmaske (1) ausbilden;
wobei die Gelenkelemente (7, 8) die Vorkammer (3) zumindest abschnittsweise umschließen;
wobei die Vorkammer (3) und der Nasenaufnahmeraum (2) jeweils an die Gasaustauschöffnung (4) unmittelbar angrenzen;
wobei eines der Gelenkelemente (7, 8) einstückig mit dem Nasenaufnahmeraum (2) verbunden ist;
wobei ein Durchmesser (d1) der Gasaustauschöffnung (4) geringer als ein Durchmesser (d2) der Vorkammer (3) ist, wobei sich die Vorkammer (3) zu der Gasaustauschöffnung (4) hin verjüngt, und wobei die Vorkammer (3) ein von dem Nasenaufnahmeraum (2) abgetrenntes Volumen aufweist.

2. Beatmungsmaske nach Anspruch 1, **dadurch gekennzeichnet, dass** die Gelenkelemente (7, 8) derart miteinander in Wirkeingriff stehen, dass diese ein Kugelgelenk (18) der Beatmungsmaske (1) ausbilden.

3. Beatmungsmaske nach Anspruch 1 oder 2, **dadurch gekennzeichnet,**
**dass** die Vorkammer (3) kugelförmig, oder ellipsoidförmig ist.

4. Beatmungsmaske nach wenigstens einem der Ansprüche 1 bis 3, **dadurch gekennzeichnet,**
**dass** die Vorkammer (3) zylinderförmig ist und die Gasaustauschöffnung (4) sowie der Gaseinlass (5) und der Gasauslass (6) jeweils an einer Stirnfläche der zylinderförmigen Vorkammer (3) angeordnet sind.

5. Beatmungsmaske nach einem der vorhergehenden Ansprüche, **dadurch gekennzeichnet,**
**dass** der Nasenaufnahmeraum (2) die Nase zumindest abschnittsweise formschlüssig umschließt.

6. Beatmungsmaske nach einem der vorhergehenden Ansprüche, **dadurch gekennzeichnet,**
**dass** die Beatmungsmaske (1) zum Befestigen derselben an dem Patienten eine Befestigungslasche (12) aufweist.

## Claims

1. Breathing mask (1) for artificially respirating a patient, said breathing mask having:
a nose receiving space (2) for receiving at least in sections the nose of the patient; and
a pre-chamber (3) that is connected by means of a gas exchange opening (4) to the nose receiving space (2) and said pre-chamber comprises a first articulated element (7) and a second articulated element (8) that is operatively connected to the first articulated element (7) and that may pivot relative to the first articulated element (7), said pre-chamber having a gas inlet (5) and a gas outlet (6) for respectively connecting to a gas flushing device; wherein one of the articulated elements (7, 8) is received and inserted in a movable manner in sections in the other articulated element (7, 8), as a result of which the articulated elements (7, 8) are in operative engagement with one another in such a manner that said articulated elements provide a moving joint (18) of the breathing mask (1) ;
wherein the articulated elements (7, 8) encompass the pre-chamber (3) at least in sections;
wherein the pre-chamber (3) and the nose receiving space (2) in each case directly adjoin the gas exchange opening (4) ;
wherein one of the articulated elements (7, 8) is connected in a single piece manner to the nose receiving space (2) ;
wherein a diameter (d1) of the gas exchange opening (4) is smaller than a diameter (d2) of the pre-chamber (3), wherein the pre-chamber (3) tapers towards the gas exchange opening (4), and wherein the pre-chamber (3) comprises a volume that is separated from the nose receiving space (2).

2. Breathing mask according to claim 1, **characterised in that** the articulated elements (7, 8) are in operative engagement with one another in such a manner that said articulated elements form a ball joint (18) of the breathing mask (1).

3. Breathing mask according to claim 1 or 2, **characterised in that** the pre-chamber (3) is ball-shaped, or ellipsoid-shaped.

4. Breathing mask according to at least one of the claims 1 to 3, **characterised in that** the pre-chamber (3) is cylinder-shaped and the gas exchange opening (4) and also the gas inlet (5) and the gas outlet (6) are arranged in each case on an end surface of the cylinder-shaped pre-chamber (3) .

5. Breathing mask according to any one of the preceding claims, **characterised in that** the nose receiving space (2) encompasses the nose at least in sections in a non-positive locking manner.

6. Breathing mask according to any one of the preceding claims, **characterised in that** the breathing mask (1) comprises a fastening link (12) for fastening said mask to the patient.

## Revendications

1. Masque respiratoire (1) permettant à un patient de respirer artificiellement, comprenant :
un espace de réception du nez (2) pour recevoir au moins par section le nez du patient ; et
une préchambre (3) reliée à l'espace de réception du nez (2) au moyen d'un orifice d'échange gazeux (4) et qui présente un premier élément d'articulation (7) et un second élément d'articulation (8) pivotant par rapport au premier élément d'articulation (7) et en liaison opérationelle avec ce premier élément d'articulation (7), avec une entrée de gaz (5) et une sortie de gaz (6) pour le branchement respectif à un moyen de circulation gazeuse ; dans lequel un des éléments d'articulation (7, 8) est reçu par section dans l'autre élément d'articulation (7, 8) et inséré de manière mobile pour permettre aux éléments d'articulation (7, 8) d'être en prise opérationelle l'un avec l'autre de manière à former une articulation mobile (18) du masque respiratoire (1) ;
dans lequel les éléments d'articulation (7, 8) entourent la préchambre (3) au moins par section ;
dans lequel la préchambre (3) et l'espace de réception du nez (2) jouxtent respectivement directement l'orifice d'échange gazeux (4) ;
dans lequel un des éléments d'articulation (7, 8) est relié d'un seul tenant à l'espace de réception du nez (2) ;
dans lequel un diamètre (d1) de l'orifice d'échange gazeux (4) est inférieur à un diamètre (d2) de la préchambre (3), la préchambre (3) rétrécissant par rapport à l'orifice d'échange gazeux (4), et la préchambre (3) présentant un volume séparé de l'espace de réception du nez (2) .

2. Masque respiratoire selon la revendication 1, **caractérisé en ce que** les éléments d'articulation (7, 8) sont en prise opérationelle l'un avec l'autre de manière à former une articulation sphérique (18) du masque respiratoire (1) .

3. Masque respiratoire selon la revendication 1 ou 2, **caractérisé en ce que** la préchambre (3) est sphérique ou ellipsoïdique.

4. Masque respiratoire selon au moins une des revendications 1 à 3, **caractérisé en ce que** la préchambre (3) est cylindrique et l'orifice d'échange gazeux (4) ainsi que l'entrée de gaz(5) et la sortie de gaz (6) sont respectivement disposés sur une surface frontale de la préchambre (3) cylindrique.

5. Masque respiratoire selon une des revendications précédentes, **caractérisé en ce que** l'espace de réception du nez (2) entoure le nez par complémentarité de forme au moins par section.

6. Masque respiratoire selon une des revendications précédentes, **caractérisé en ce que** le masque respiratoire (1) présente une patte de fixation (12) pour fixer celui-ci à un patient.
